# EUROPEAN PATENT SPECIFICATION

(11) **EP 4 735 915 B1**
(45) Date of publication and mention of the grant of the patent: **04.11.2026**
(21) Application number: 24733205.9
(22) Date of filing: 17.06.2024
(51) Int. Cl.: G01T 1/29, A61B 6/03

(54) **MODULAR COMPUTED TOMOGRAPHY DETECTOR CONFIGURATION**
MODULARE COMPUTERTOMOGRAPHIEDETEKTORKONFIGURATION
CONFIGURATION MODULAIRE DE DÉTECTEUR DE TOMODENSITOMÉTRIE

(30) Priority: 27.06.2023 EP 23181594
(43) Date of publication of application: 06.05.2026
(73) Proprietor: Koninklijke Philips N.V., 5656 AE Eindhoven (NL)
(72) Inventor: KOEHLER, Thomas, 5656 AG Eindhoven (NL); VOGTMEIER, Gereon, 5656 AG Eindhoven (NL); JOHNSON, Mark Thomas, 5656 AG Eindhoven (NL); WEISS, Steffen, 5656 AG Eindhoven (NL)
(74) Representative: Philips Intellectual Property & Standards
(86) International application number: PCT/EP2024/066784
(87) International publication number: WO 2025/002880

(56) References cited:
- CN-A- 104 983 439
- JP-A- H03 279 887
- US-A1- 2020 323 500
- US-B1- 7 433 443

## Description

### FIELD OF THE INVENTION

The present invention relates to a detection system for a computed tomography system, a computed tomography system comprising the detection system, and a method of imaging a subject with the computed tomography system.

### BACKGROUND OF THE INVENTION

Today computed tomography CT imaging systems are quite expensive due to the large detector area that has to be provided due to the required coverage in both, the direction of the longitudinal extension of the patient to be imaged and the direction of the rotational scanning movement of the CT system. However, not in all cases a large detector is necessary and, depending on the application, a smaller field of view of the detection system, i.e. the extension of the detector in the rotation direction, may be acceptable. At the same time, a larger coverage in patient direction, i.e. the longitudinal axis of the scanning movement of the CT scanner, either allows for a faster scan to avoid movement artifacts and also reduces total scan time and/or allows for scanning of dynamic events.

CN 104 983 439 A describes a computed tomography scanner including a rotating disc provided with a plurality of flat panel detectors.

US 2020/323500 A1 describes a medical imaging system including a detector configured to detect radiation that has passed a collimator, wherein the detector includes a plurality of detector tiles and at least one detector tile is movebale with respect to other detector tiles.

JP H03 279887 A describes a nuclear medicine apparatus including a plurality of radiation detectors arranged adjacent to each other at an angle with respect to their detection surfaces.

The inventors of the present invention have thus found that it would be advantageous to have an improved detection system for a computed tomography system that at least partially solves these problems and allows an adjustment of the detection area of the computed tomography system in order to adapt to the needs of the specific imaging procedure.

### SUMMARY OF THE INVENTION

It is an object of the present invention to provide an improved detection system for a computed tomography system that allows flexible adaption of the detection area to the needs of a specific imaging procedure.

The object of the present invention is solved by the subject matter of the independent claims, wherein further embodiments are incorporated in the dependent claims.

The described embodiments similarly pertain to the detection system for a computed tomography system, the computed tomography system comprising the detection system, and the method of imaging a subject with the computed tomography system. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a detection system for a computed tomography system. The detection system comprises a first detector configured for detecting X-ray radiation of the computed tomography system impinging onto the first detector, and a second detector configured for detecting X-ray radiation of the computed tomography system impinging onto the second detector. The detection system further comprises a repositioning mechanism configured for repositioning at least one of the first detector and the second detector with respect to the other one of the first detector and the second detector. The repositioning mechanism comprises a guiding mechanism configured for guiding the at least one of the first detector and the second detector along a predetermined track from a first position to a second position, and/or the repositioning mechanism comprises a rotating mechanism configured for rotating the at least one of the first detector and the second detector with respect to a rotational axis essentially parallel to a direction of the X-ray radiation. The first detector and/or the second detector comprises an anti-scatter grid configured for suppressing scattered X-ray radiation reaching the respective detector. The anti-scatter grid is configured for aligning a focus of the anti-scatter grid with respect to an X-ray source of the computed tomography system. The anti-scatter grid is a one-dimensional anti-scatter grid and is configured for adjusting a direction of grid lines of the one-dimensional anti-scatter grid with respect to an aspect ratio of a detection area of the computed tomography system.

Thus, a modular concept for reconfigurable detector sub-areas that can be moved to different positions is provided. The detection system can thus be adapted to different required area coverages as detector sub-areas may be moved to different positions. Different geometry setups of the detection system can be used for imaging and flexibility in positioning of the detection system with different configurations is provided. Different geometry settings of the detection area of the computed tomography system can be preferred for different applications. For example, for brain scans, pediatric imaging or extremity imaging, the complete field of view of the detection system in the rotation direction of the computed tomography scanner may be not required as the object to be imaged can be smaller in size than the maximum field of view of the detection system. However, increasing the extension of the coverage of the detection system in the patient direction may be quite interesting, as doubling the detector coverage in this direction allows to cut the acquisition time in half, which may be very important for non-compliant patients such as children or patients with stroke, for example. In another imaging setup, a larger field of view in the rotational direction may be required, due to the size of the patient or the required imaging area, for example, while a large coverage of the detection system in the longitudinal axis of the patient may not be necessary and could be compensated for by a longer acquisition time with additional rotational scanning movements around the z-axis of the computed tomography scanner.

Therefore, a large detector may solve many of the aforementioned problems but may be expensive. A modular reconfigurable detector according to the invention can be adapted to the different required area coverages and aspect ratios of the detection area and allows a good trade-off between price and the required area coverage.

Besides the pure size of the detection area, also the type of a detector could be attractive for trade off configurations. Thus, the different movable detector modules can represent different types of detector. This could be, e.g., a spectral detector that can be used in the center region of the detection area and/or can be moved to the side region, or a spectral detector that covers the complete field of view in the rotation direction but may be of smaller size in the longitudinal patient direction.

In an embodiment of the invention, the first detector and the second detector are configured for forming a detection area of the computed tomography system.

In an embodiment of the invention, the repositioning mechanism is configured for adjusting an aspect ratio of the detection area of the computed tomography system.

In an embodiment of the invention, the first detector and the second detector are configured for forming the detection area essentially without a gap in-between the first detector and the second detector.

In an embodiment of the invention, the repositioning mechanism comprises a locking mechanism configured for locking in place the at least one of the first detector and the second detector in at least one of the first position and the second position.

In an embodiment of the invention, the repositioning mechanism is configured for adjusting an angular tilt of the first detector and/or the second detector with respect to a direction of the X-ray radiation of the computed tomography system.

In an embodiment of the invention, the second detector differs from the first detector with respect to a spectral response to the X-ray radiation.

In an embodiment of the invention, the detection system comprises at least one third detector configured for detecting X-ray radiation of the computed tomography system impinging onto the third detector, and the repositioning mechanism is configured for repositioning the at least one third detector with respect to the first detector and the second detector.

According to another aspect of the invention, there is provided a computed tomography system. The computed tomography system comprises the detection system according to any of the preceding embodiments, an X-ray source configured for emitting X-ray radiation, and a collimator configured for collimating the X-ray radiation to a detection area formed by the first detector and the second detector.

According to another aspect of the invention, there is provided a method of imaging a subject with a computed tomography system according to the preceding embodiment. The method comprises the steps of providing a computed tomography system according to the preceding embodiment, determining a detection area for imaging the subject, repositioning at least one of the first detector and the second detector with respect to the other one of the first detector and the second detector to form the determined detection area, and imaging the subject.

In an embodiment of the invention, the method comprises the step of locking in place the at least one of the first detector and the second detector after the step of repositioning the at least one of the first detector and the second detector.

In an embodiment of the invention, the method comprises the step of adjusting an angular tilt of the first detector and/or the second detector with respect to a direction of the X-ray radiation of the computed tomography system.

In an embodiment of the invention, the method is performed automatically based on an imaging protocol and/or a patient geometry. Thus, the workflow can be improved.
Thus, the benefits provided by any of the above aspects equally apply to all of the other aspects and vice versa.

In summary, the invention relates to a detection system for a computed tomography system. The detection system comprises a first detector and a second detector, each detector being configured for detecting X-ray radiation of the computed tomography system impinging onto the respective detector. The detection system further comprises a repositioning mechanism configured for repositioning at least one of the first detector and the second detector with respect to the other one of the first detector and the second detector.

One of the advantages of embodiments of the present invention of the detection system for a computed tomography system is that a modular reconfigurable detector can be adapted to the different required area coverages of the detector and thus allows a good trade-off between price and coverage. Another advantage is that a field of view of the detection system can be extended. Another advantage is that besides the pure size and aspect ratio of the detector, also the type of the detector and the configuration of an anti-scatter grid can be adjusted in accordance to the needs of the specific imaging procedure.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic setup of a standard detection system with fixed aspect ratio for a computed tomography system.
Fig. 2 shows a schematic setup of a detection system for a computed tomography system according to an embodiment of the invention.
Fig. 3 shows a schematic setup of a detection system for a computed tomography system according to another embodiment of the invention.
Figs. 4A and 4B show a schematic setup of a detection system for a computed tomography system comprising a one-dimensional anti-scatter grid according to the invention.
Fig. 5 shows a schematic setup of a computed tomography system comprising the detection system according to another embodiment of the invention.
Fig. 6 shows a block diagram of a method of imaging a subject with a computed tomography system according to an embodiment of the invention.

### DETAILED DESCRIPTION OF EMBODIMENTS

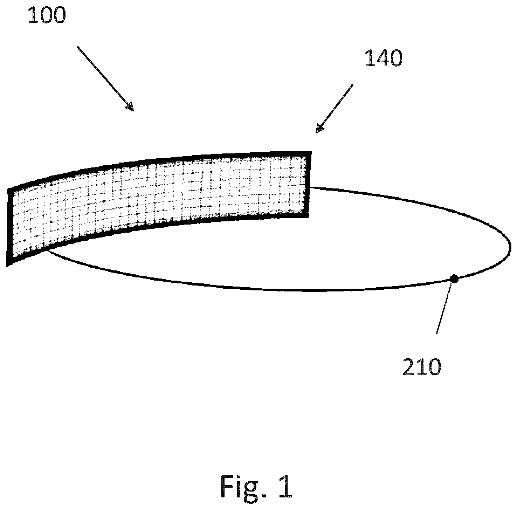

Fig. 1 shows a schematic setup of a standard detection system with fixed aspect ratio for a computed tomography system. For general purpose systems, the fan angle may be large enough to support object sizes in the order of 500 mm diameter. However, this fixed geometry setup is not adaptable to the needs of a specific imaging procedure, where a different aspect ratio of the detection area may be desired.

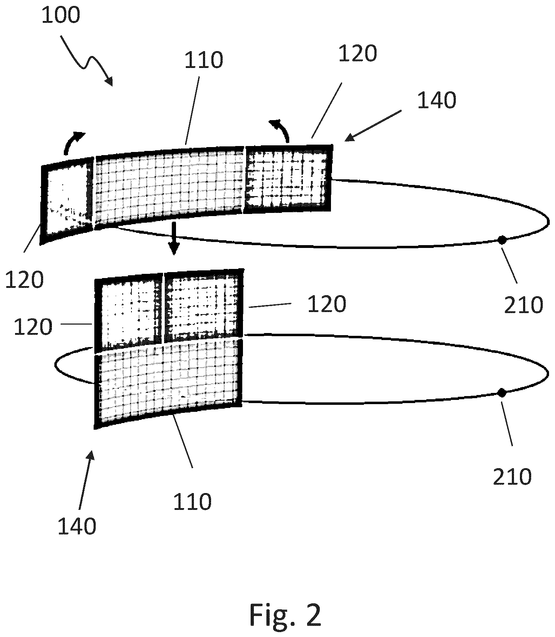

Fig. 2 shows a schematic setup of a detection system 100 for a computed tomography system 200 according to an embodiment of the invention. A modular concept for detector sub-areas that could be moved to different positions is shown. Different geometry setups provide flexibility in positioning with several configurations. Thus, different geometry settings can preferably be provided for different applications. For example, for brain scans, pediatric imaging and extremity imaging the complete field of view may be not required as the object may be smaller than the field of view. However, regarding the extension of the detection system 100 in patient direction, a doubling the detector coverage allows to cut the acquisition time in half, which may be very important for non-compliant patients, for example.

Therefore, with reference to Fig. 2, different geometries of the same type of detector are illustrated. The detection system 100 comprises a first detector 110 and a second detector 120, each detector being configured for detecting X-ray radiation of the computed tomography system 200 impinging onto the respective detector. In this embodiment, two second detectors 120 are shown. The first detector 110 and the second detectors 120 together form a detection area 140, which in this case is broad in rotation direction from left to right, but small in the patient direction from top to bottom. On the opposite side on the path of the scanning movement of the computed tomography scanner 200, an X-ray source 210 of the computed tomography scanner 200 is indicated. The second detectors 120 can be relocated by means of a repositioning mechanism 130, which is not shown in Fig. 2.

After repositioning the second detectors 120, they form together with the first detector 110 a detection area 140 a different aspect ratio than before, the new detection area 140 in this exemplary embodiment with a nearly quadratic aspect ratio, which is shown in the lower part of Fig. 2.

With a repositioning mechanism 130, the second detector 120 can be relocated with respect to the first detector 110 to adjust the aspect ratio of the detection area 140. The repositioning mechanism 130 comprises a guiding mechanism for guiding the at least one of the first detector 110 and the second detector 120 along a predetermined track from a first position to a second position. In addition or alternatively, the repositioning mechanism 130 comprises a rotating mechanism for rotating the at least one of the first detector 110 and the second detector 120 with respect to a rotational axis essentially parallel to a direction of the X-ray radiation. Space for rotation of one of the first or second detectors can be provided by temporarily elevating the respective detector element towards an isocenter for rotation of the computer tomography system, for example.

The repositioning mechanism can comprise a locking mechanism for locking in place the at least one of the first detector 110 and the second detector 120 in at least one of the first position and the second position. Thus, a reliable and stable fixation of the respective detectors between the required end positions and a robust movement mechanism can be provided, which can be a rail-like system, which may be hydraulic with defined end-positions with fixtures and respective movement actuator elements. Special alignment features may be used at the different interfacing sides of the modules to get a stable and precise position at both end positions. Small alignment pins fitting into a cone hole or step like interface ensure same height and z/phi position of the detectors.

The repositioning mechanism 130 is preferably configured for adjusting an angular tilt of the first detector 110 and/or the second detector 120 with respect to a direction of the X-ray radiation of the computed tomography system 200. Tilting can be performed with respect to an optical axis of the system, which may be defined as the line from the X-ray source to the rotation axis of the computed tomography system that lies within the plane of rotation. Hardware adaptations and software adaptions to the detection system 100 and the computed tomography apparatus 200 for tiling of modules may be necessary, including adaption of the X-ray beam collimation. For each possible position setting of the detectors, a calibration of the detailed detector location can be done and the data may be considered for the image processing and reconstruction.

Preferably, the second detector 120 may be different from the first detector 110 with respect to a spectral response to the X-ray radiation. The sub-detector modules may be different types of detectors. This could be a standard integrating computed tomography detector, a dual layer spectral computed tomography detector, or a spectral photon counting detector. With such a setup the more expensive spectral detectors could be positioned according to the region of interest.

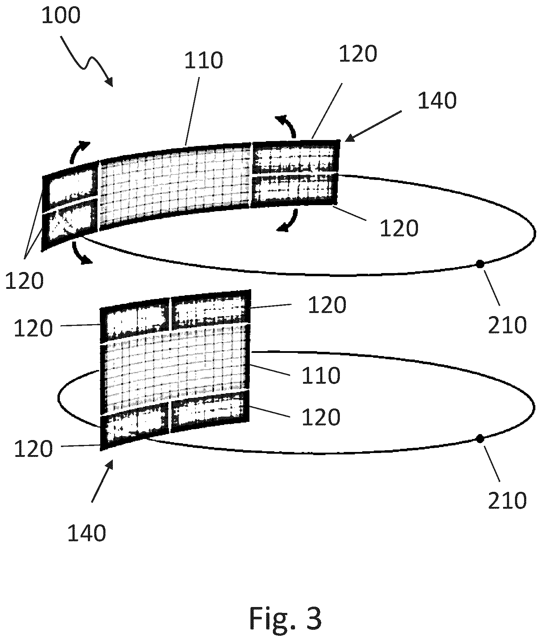

With reference to Fig. 3, a schematic setup of a detection system 100 for a computed tomography system 200 according to another embodiment of the invention is shown. In this embodiment, four second detectors 120 are shown, which are repositionable and form together with the first detector 110 the detection area 140. Depending on the size of the sub-detector, additional options for the geometry settings are possible. The smaller sub-detector modules allow for more flexible distribution and avoid the need to move the central part along the z-axis after repositioning of the detectors. By repositioning the at least one of the first detector 110 or the second detector 120 with respect to the other detectors, the aspect ratio of the detection area 140 can be adjusted. On top of Fig. 3, a small and long detection area 140 is shown, whereas after repositioning on the bottom of Fig. 2 a detection area with different aspect ratio is shown. The different trajectories for the movement of an actuator are indicated with arrows. Preferably, the first detector 110 and the second detector 120 form the detection area 140 essentially without a gap in-between the first detector 110 and the second detector 120 in order to avoid dead spaces in the detection area 140.

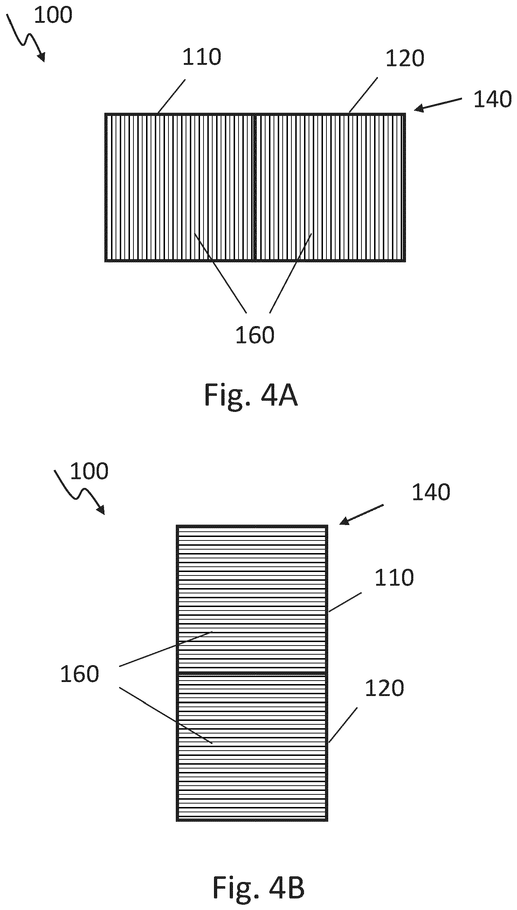

Figs. 4A and 4B show a schematic setup of a detection system 100 for a computed tomography system 200 comprising a one-dimensional anti-scatter grid 160 according to the invention. At least one of the first detector 110 and the second detector 120 comprises an anti-scatter grid 160 configured for suppressing scattered X-ray radiation reaching the respective detector. With an adjustable anti-scatter grid 160, a focus of the anti-scatter grid 160 with respect to an X-ray source 210 of the computed tomography system 200 can be adjusted.

An adaptation of the focal spot alignment for the anti-scatter grids (ASGs) and the collimation of the X-ray beam matching to the detection area 140 is provided. The correct angular tilt of the respective detector with an ASG in both end-positions of the detector trajectory before and after repositioning is provided. This may be always the focused direction from the focal spot position of the X-ray source 210 to the channels of the ASG and the detector element. Alternatively, individual sub-module focus alignment of the anti-scatter grid might be an alternative method compared to the angular tilt of the complete detector module comprising the anti-scatter grid.

The anti-scatter grid 160 is a one-dimensional anti-scatter grid, and the anti-scatter grid is configured for adjusting a direction of grid lines of the one-dimensional anti-scatter grid with respect to an aspect ratio of the detection area 140 of the computed tomography system 200. Note, however, that computed tomography systems with moderate collimation are usually equipped with one-dimensional anti-scatter grids, i.e., they comprise only lamellae in one direction. Focusing of such type of collimators can be easier as tilting may not be required. Therefore, one-dimensional anti-scatter grids are used. One-dimensional anti-scatter grids are typically used for detectors up to, e.g. 64 lines, as for larger patient coverage the scatter becomes larger and two-dimensional anti-scatter grids are preferred, e.g. for a scanner with 128 lines. In case of a detector configuration where the detector size is larger in patient direction, good scatter rejection may be necessary, e.g. by rotating the one-dimensional anti-scatter grid in the respective direction.

Detector modules like the first detector 110 or the second detector 120 are equipped with a one-dimensional anti-scatter grid. It is preferably proposed to rotate these grids or detectors modules such that the grid lines are either parallel to the patient direction or to the angular direction. For scanning a long and thin body part extending along the patient direction, the X-ray beam of the X-ray source 210 may be collimated by a collimator 220 of the computed tomography system 200 narrow in the angular direction and wide in the patient direction to cover this body part. In that case, the grid lines should be oriented in angular direction to minimize a scattering signal. In case of scanning a wide patient but with limited field of view in the direction of the patient, the beam may be collimated narrow in that direction and wide in angular direction. In that case, the grid lines should be oriented in patient direction to minimize the scattering signal. For optimal orientation of the grids towards the focal spot for all module positions and rotations, the modules positioned not centrally at z=0 but further off in patient direction might need to be tilted towards the focal spot of the X-ray source, e.g., by shaping a base structure on which the detector modules are repositionably fixed. This shape of the base structure may essentially form a sphere made of tiles, where each tile has the format of a detector module. Tuning of the angular alignment to the focal spot can be adapted for each detector position.

Preferably, detector modules with a one-dimensional anti-scatter grid and detector modules with a two-dimensional anti-scatter grid may be mixed in one detection system. While one-dimensional anti-scatter grid may be cheaper, two-dimensional anti-scatter grids can better suppress scattering and improve image quality. Mixing these allows to set the trade-off between cost and image quality. Especially for the modular side modules, a two-dimensional anti-scatter grid would be beneficial as they can be located in a z-position where most cross scatter can have image degrading influence.

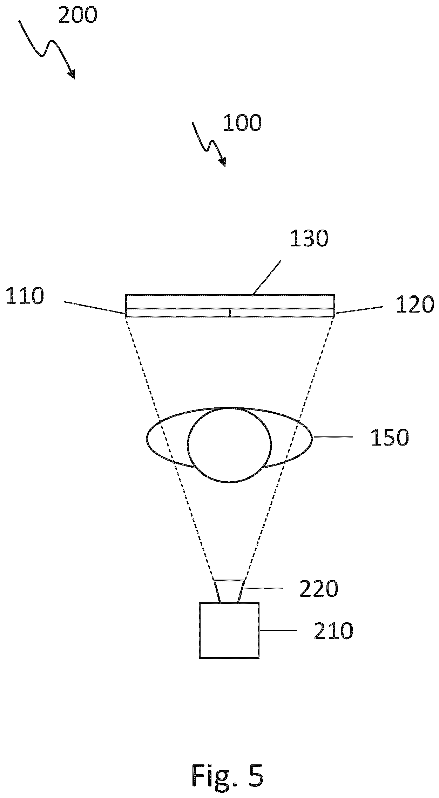

Fig. 5 shows a schematic setup of a computed tomography system 200 comprising the detection system 100 according to another embodiment of the invention. The computed tomography system 200 comprises an X-ray source 210 and a collimator 220. The subject 150 is irradiated and the radiation is detected on the detection area 140 formed by the first detector 110 and the second detector 120. A repositioning mechanism 130 is provided for repositioning at least one of the first detector 110 and the second detector 120 with respect to the other one of the first detector 110 and the second detector 120.

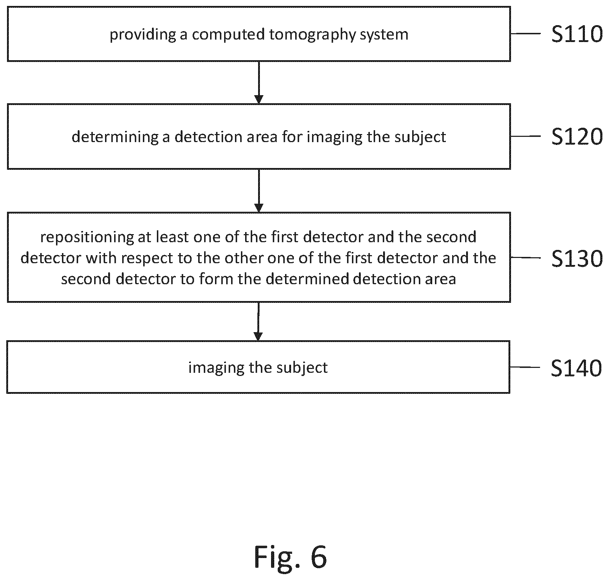

Fig. 6 shows a block diagram of a method of imaging a subject 150 with a computed tomography system 200 according to an embodiment of the invention. The method comprises the step S110 of providing a computed tomography system according to any of the preceding embodiments, and the step S120 of determining a detection area 140 for imaging the subject. The method comprises further the step S130 of repositioning at least one of the first detector 110 and the second detector 120 with respect to the other one of the first detector 110 and the second detector 120 to form the determined detection area 140, and the step S140 of imaging the subject.

Preferably, the method comprises the step of locking in place the at least one of the first detector 110 and the second detector 120 after the step of repositioning the at least one of the first detector 110 and the second detector 120, and/or the step of adjusting an angular tilt of the first detector 110 and/or the second detector 120 with respect to a direction of the X-ray radiation of the computed tomography system 200.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: detection system
- 110: first detector
- 120: second detector
- 130: repositioning mechanism
- 140: detection area
- 150: subject
- 160: anti-scatter grid
- 200: computed tomography imaging system
- 210: X-ray source
- 220: collimator

## Claims

1. A detection system (100) for a computed tomography system (200), the detection system comprising
a first detector (110) configured for detecting X-ray radiation of the computed tomography system (200) impinging onto the first detector (110);
a second detector (120) configured for detecting X-ray radiation of the computed tomography system (200) impinging onto the second detector (120); and
a repositioning mechanism (130) configured for repositioning at least one of the first detector (110) and the second detector (120) with respect to the other one of the first detector (110) and the second detector (120);
wherein the first detector (110) and/or the second detector (120) comprises an anti-scatter grid (160) configured for suppressing scattered X-ray radiation reaching the respective detector;
**characterized in that**
the repositioning mechanism (130) comprises a guiding mechanism configured for guiding the at least one of the first detector (110) and the second detector (120) along a predetermined track from a first position to a second position, and/or wherein the repositioning mechanism (130) comprises a rotating mechanism configured for rotating the at least one of the first detector (110) and the second detector (120) with respect to a rotational axis essentially parallel to a direction of the X-ray radiation;
wherein the anti-scatter grid (160) is configured for aligning a focus of the anti-scatter grid (160) with respect to an X-ray source (210) of the computed tomography system (200); and
wherein the anti-scatter grid (160) is a one-dimensional anti-scatter grid, and wherein the anti-scatter grid (160) is configured for adjusting a direction of grid lines of the one-dimensional anti-scatter grid with respect to an aspect ratio of a detection area (140) of the computed tomography system (200).

2. The detection system (100) according to claim 1, wherein the first detector (110) and the second detector (120) are configured for forming a detection area (140) of the computed tomography system (200).

3. The detection system (100) according to claim 2, wherein the repositioning mechanism (130) is configured for adjusting an aspect ratio of the detection area (140) of the computed tomography system (200).

4. The detection system (100) according to any of claims 2 to 3, wherein the first detector (110) and the second detector (120) are configured for forming the detection area (140) essentially without a gap in-between the first detector (110) and the second detector (120).

5. The detection system (100) according to claim 1, wherein the repositioning mechanism (130) comprises a locking mechanism configured for locking in place the at least one of the first detector (110) and the second detector (120) in at least one of the first position and the second position.

6. The detection system (100) according to any of the preceding claims, wherein the repositioning mechanism (130) is configured for adjusting an angular tilt of the first detector (110) and/or the second detector (120) with respect to a direction of the X-ray radiation of the computed tomography system (200).

7. The detection system (100) according to any of the preceding claims, wherein the second detector (120) differs from the first detector (110) with respect to a spectral response to the X-ray radiation.

8. The detection system (100) according to any of the preceding claims, wherein the detection system (100) comprises at least one third detector configured for detecting X-ray radiation of the computed tomography system (200) impinging onto the third detector, and wherein the repositioning mechanism (130) is configured for repositioning the at least one third detector with respect to the first detector (110) and the second detector (120).

9. A computed tomography system (200), the computed tomography system comprising
the detection system (100) according to any of claims 1 to 8;
an X-ray source (210) configured for emitting X-ray radiation; and
a collimator (220) configured for collimating the X-ray radiation to a detection area (140) formed by the first detector (110) and the second detector (120).

10. A method of imaging a subject (150) with a computed tomography system (200) according to claim 9, the method comprising the steps of:
providing (S110) a computed tomography system (200) according to claim 9;
determining (S120) a detection area (140) for imaging the subject (150);
repositioning (S130) at least one of the first detector (110) and the second detector (120) with respect to the other one of the first detector (110) and the second detector (120) to form the determined detection area (140); and
imaging (S140) the subject (150).

11. The method according to claim 10, wherein the method comprises the step of locking in place the at least one of the first detector (110) and the second detector (120) after the step of repositioning the at least one of the first detector (110) and the second detector (120).

12. The method according to any of claims 10 or 11, wherein the method comprises the step of adjusting an angular tilt of the first detector (110) and/or the second detector (120) with respect to a direction of the X-ray radiation of the computed tomography system (200).

## Patentansprüche

1. Detektionssystem (100) für ein Computertomographiesystem (200), wobei das Detektionssystem umfasst:
einen ersten Detektor (110), der dazu ausgelegt ist, auf den ersten Detektor (110) auftreffende Röntgenstrahlung des Computertomographiesystems (200) zu detektieren;
einen zweiten Detektor (120), der dazu ausgelegt ist, auf den zweiten Detektor (120) auftreffende Röntgenstrahlung des Computertomographiesystems (200) zu detektieren; und
einen Neupositionierungsmechanismus (130), der dazu ausgelegt ist, den ersten Detektor (110) und/oder den zweiten Detektor (120) relativ zum jeweils anderen des ersten Detektors (110) und des zweiten Detektors (120) neu zu positionieren;
wobei der erste Detektor (110) und/oder der zweite Detektor (120) ein Antistreugitter (160) umfasst, das dazu ausgelegt ist, gestreute Röntgenstrahlung zu unterdrücken, die den jeweiligen Detektor erreicht;
**dadurch gekennzeichnet, dass** der Neupositionierungsmechanismus (130) einen Führungsmechanismus umfasst, der dazu ausgelegt ist, den ersten Detektor (110) und/oder den zweiten Detektor (120) entlang einer vorbestimmten Bahn von einer ersten Position zu einer zweiten Position zu führen, und/oder wobei der Neupositionierungsmechanismus (130) einen Drehmechanismus umfasst, der dazu ausgelegt ist, den ersten Detektor (110) und/oder den zweiten Detektor (120) in Bezug auf eine Drehachse zu drehen, die im Wesentlichen parallel zu einer Richtung der Röntgenstrahlung verläuft;
wobei das Antistreugitter (160) dazu ausgelegt ist, einen Fokus des Antistreugitters (160) in Bezug auf eine Röntgenquelle (210) des Computertomographiesystems (200) auszurichten; und
wobei das Antistreugitter (160) ein eindimensionales Antistreugitter ist und wobei das Antistreugitter (160) dazu ausgelegt ist, eine Richtung von Gitterlinien des eindimensionalen Antistreugitters in Bezug auf ein Seitenverhältnis eines Detektionsbereichs (140) des Computertomographiesystems (200) anzupassen.

2. Detektionssystem (100) nach Anspruch 1, wobei der erste Detektor (110) und der zweite Detektor (120) dazu ausgelegt sind, einen Detektionsbereich (140) des Computertomographiesystems (200) zu bilden.

3. Detektionssystem (100) nach Anspruch 2, wobei der Neupositionierungsmechanismus (130) dazu ausgelegt ist, ein Seitenverhältnis des Detektionsbereichs (140) des Computertomographiesystems (200) anzupassen.

4. Detektionssystem (100) nach einem der Ansprüche 2 bis 3, wobei der erste Detektor (110) und der zweite Detektor (120) dazu ausgelegt sind, den Detektionsbereich (140) im Wesentlichen ohne Zwischenraum zwischen dem ersten Detektor (110) und dem zweiten Detektor (120) zu bilden.

5. Detektionssystem (100) nach Anspruch 1, wobei der Neupositionierungsmechanismus (130) einen Verriegelungsmechanismus umfasst, der dazu ausgelegt ist, den ersten Detektor (110) und/oder den zweiten Detektor (120) in der ersten Position und/oder der zweiten Position zu verriegeln.

6. Detektionssystem (100) nach einem der vorhergehenden Ansprüche, wobei der Neupositionierungsmechanismus (130) dazu ausgelegt ist, eine Winkelneigung des ersten Detektors (110) und/oder des zweiten Detektors (120) in Bezug auf eine Richtung der Röntgenstrahlung des Computertomographiesystems (200) anzupassen.

7. Detektionssystem (100) nach einem der vorhergehenden Ansprüche, wobei sich der zweite Detektor (120) hinsichtlich einer spektralen Empfindlichkeit gegenüber der Röntgenstrahlung vom ersten Detektor (110) unterscheidet.

8. Detektionssystem (100) nach einem der vorhergehenden Ansprüche, wobei das Detektionssystem (100) mindestens einen dritten Detektor umfasst, der dazu ausgelegt ist, auf den dritten Detektor auftreffende Röntgenstrahlung des Computertomographiesystems (200) zu detektieren, und wobei der Neupositionierungsmechanismus (130) dazu ausgelegt ist, den mindestens einen dritten Detektor relativ zum ersten Detektor (110) und zum zweiten Detektor (120) neu zu positionieren.

9. Computertomographiesystem (200), wobei das Computertomographiesystem umfasst:
das Detektionssystem (100) nach einem der Ansprüche 1 bis 8;
eine Röntgenquelle (210), die zum Aussenden von Röntgenstrahlung ausgelegt ist; und
einen Kollimator (220), der zum Kollimieren der Röntgenstrahlung auf einen Detektionsbereich (140) ausgelegt ist, der durch den ersten Detektor (110) und den zweiten Detektor (120) gebildet wird.

10. Verfahren zum bildgebenden Darstellen eines Patienten (150) mit einem Computertomographiesystem (200) nach Anspruch 9, wobei das Verfahren die folgenden Schritte umfasst:
Bereitstellen (S110) eines Computertomographiesystems (200) nach Anspruch 9;
Bestimmen (S120) eines Detektionsbereichs (140) zum bildgebenden Darstellen des Patienten (150);
Neupositionieren (S130) des ersten Detektors (110) und/oder des zweiten Detektors (120) in Bezug auf den jeweils anderen des ersten Detektors (110) und des zweiten Detektors (120), um den bestimmten Detektionsbereich (140) zu bilden; und
bildgebendes Darstellen (S140) des Patienten (150).

11. Verfahren nach Anspruch 10, wobei das Verfahren den Schritt des Verriegelns des ersten Detektors (110) und/oder des zweiten Detektors (120) nach dem Schritt des Neupositionierens des ersten Detektors (110) und/oder des zweiten Detektors (120) umfasst.

12. Verfahren nach einem der Ansprüche 10 oder 11, wobei das Verfahren den Schritt des Anpassens einer Winkelneigung des ersten Detektors (110) und/oder des zweiten Detektors (120) in Bezug auf eine Richtung der Röntgenstrahlung des Computertomographiesystems (200) umfasst.

## Revendications

1. Système (100) de détection d'un système (200) de tomodensitométrie, le système de détection comprenant un premier détecteur (110) configuré pour détecter un rayonnement X du système (200) de tomodensitométrie frappant le premier détecteur (110) ;
un deuxième détecteur (120) configuré pour détecter un rayonnement X du système (200) de tomodensitométrie frappant le deuxième détecteur (120) ; et
un mécanisme (130) de repositionnement configuré pour repositionner au moins un détecteur parmi le premier détecteur (110) et le deuxième détecteur (120) par rapport à l'autre détecteur parmi le premier détecteur (110) et le deuxième détecteur (120) ;
le premier détecteur (110) et/ou le deuxième détecteur (120) comprenant une grille antidiffusion (160) configurée pour limiter le rayonnement X diffusé atteignant le détecteur respectif ;
**caractérisé en ce que** le mécanisme (130) de repositionnement comprend un mécanisme de guidage configuré pour guider le ou les détecteurs considérés parmi le premier détecteur (110) et du deuxième détecteur (120) le long d'une piste prédéterminée d'une première position à une deuxième position, et/ou dans lequel le mécanisme (130) de repositionnement comprend un mécanisme de rotation configuré pour faire tourner le ou les détecteurs considérés parmi le premier détecteur (110) et le deuxième détecteur (120) par rapport à un axe de rotation essentiellement parallèle à une direction du rayonnement X ;
la grille antidiffusion (160) étant configurée pour aligner un foyer de la grille antidiffusion (160) par rapport à une source (210) de rayons X du système (200) de tomodensitométrie ; et
la grille antidiffusion (160) étant une grille antidiffusion unidimensionnelle, et la grille antidiffusion (160) étant configurée pour ajuster une direction des lignes de grille de la grille antidiffusion unidimensionnelle par rapport à un facteur de forme d'une zone (140) de détection du système (200) de tomodensitométrie.

2. Système (100) de détection selon la revendication 1, le premier détecteur (110) et le deuxième détecteur (120) étant configurés pour former une zone (140) de détection du système (200) de tomodensitométrie.

3. Système (100) de détection selon la revendication 2, le mécanisme (130) de repositionnement étant configuré pour ajuster un facteur de forme de la zone (140) de détection du système (200) de tomodensitométrie.

4. Système (100) de détection selon l'une quelconque des revendications 2 à 3, le premier détecteur (110) et le deuxième détecteur (120) étant configurés pour former la zone (140) de détection essentiellement sans écartement entre le premier détecteur (110) et le deuxième détecteur (120).

5. Système (100) de détection selon la revendication 1, le mécanisme (130) de repositionnement comprenant un mécanisme de verrouillage configuré pour verrouiller en place le ou les détecteurs considérés parmi le premier détecteur (110) et le deuxième détecteur (120) dans la première position et/ou la deuxième position.

6. Système (100) de détection selon l'une quelconque des revendications précédentes, le mécanisme (130) de repositionnement étant configuré pour régler une inclinaison angulaire du premier détecteur (110) et/ou du deuxième détecteur (120) par rapport à une direction du rayonnement X du système (200) de tomodensitométrie.

7. Système (100) de détection selon l'une quelconque des revendications précédentes, dans lequel le deuxième détecteur (120) diffère du premier détecteur (110) en ce qui concerne une réponse spectrale au rayonnement X.

8. Système (100) de détection selon l'une quelconque des revendications précédentes, le système (100) de détection comprenant au moins un troisième détecteur configuré pour détecter un rayonnement X du système (200) de tomodensitométrie frappant le troisième détecteur, et le mécanisme (130) de repositionnement étant configuré pour repositionner le ou les troisièmes détecteurs par rapport au premier détecteur (110) et au deuxième détecteur (120).

9. Système (200) de tomodensitométrie, le système de tomodensitométrie comprenant
le système (100) de détection selon l'une quelconque des revendications 1 à 8 ;
une source (210) de rayons X configurée pour émettre un rayonnement X ; et
un collimateur (220) configuré pour collimater le rayonnement X sur une zone (140) de détection formée par le premier détecteur (110) et le deuxième détecteur (120).

10. Procédé d'imagerie d'un sujet (150) avec un système (200) de tomodensitométrie selon la revendication 9, le procédé comprenant les étapes de :
mise en place (S110) d'un système (200) de tomodensitométrie selon la revendication 9 ;
détermination (S120) d'une zone (140) de détection pour imager le sujet (150) ;
repositionnement (S130) d'au moins un détecteur parmi le premier détecteur (110) et le deuxième détecteur (120) par rapport à l'autre parmi le premier détecteur (110) et le deuxième détecteur (120) pour former la zone (140) de détection déterminée ; et
imagerie (S140) du sujet (150).

11. Procédé selon la revendication 10, le procédé comprenant l'étape de verrouillage en place du ou des détecteurs considérés parmi le premier détecteur (110) et le deuxième détecteur (120) après l'étape de repositionnement du ou des détecteurs considérés parmi le premier détecteur (110) et le deuxième détecteur (120).

12. Procédé selon l'une quelconque des revendications 10 ou 11, le procédé comprenant l'étape d'ajustement d'une inclinaison angulaire du premier détecteur (110) et/ou du deuxième détecteur (120) par rapport à une direction du rayonnement X du système (200) de tomodensitométrie.
